## Europäisches Patentamt
## European Patent Office
## Office européen des brevets

(19)

(11) Publication number: **0 173 243**
**B1**

(12)

# EUROPEAN PATENT SPECIFICATION

(45) Date of publication of patent specification: **16.01.91**

(51) Int. Cl.⁵: **G 01 P 5/00, A 61 B 8/06**

(21) Application number: **85110505.6**

(22) Date of filing: **23.08.85**

(54) **Apparatus for measuring the velocity of a stream of particles.**

(30) Priority: **28.08.84 US 619881**

(43) Date of publication of application:
**05.03.86 Bulletin 86/10**

(45) Publication of the grant of the patent:
**16.01.91 Bulletin 91/03**

(84) Designated Contracting States:
**DE FR GB IT**

(56) References cited:
**EP-A-0 014 793**
**GB-A-2 059 590**

(73) Proprietor: **Hewlett-Packard Company**
**Mail Stop 20 B-O, 3000 Hanover Street**
**Palo Alto, California 94304 (US)**

(72) Inventor: **Tykulsky, Alexander**
**Carlisle 454 Heald Road**
**Massachusetts 01741 (US)**
Inventor: **Thiele, Karl E.**
**49 North Avenue**
**Melrose Massachusetts 02176 (US)**
Inventor: **Halberg, Leslie T.**
**27 Rockland Avenue**
**Malden Massachusetts 02148 (US)**

(74) Representative: **Schoppe, Fritz, Dipl.-Ing.**
**Seitnerstrasse 42**
**D-8023 Pullach bei München (DE)**

Courier Press, Leamington Spa, England.

**Description**

The invention relates to an ultrasonic pulsed Doppler system in accordance with the preamble of claim 1 and to a method for measuring the velocity of particles flowing through a sample volume by means of an ultrasonic pulsed Doppler system in accordance with the preamble of claim 7.

Such a system and method are used, for example, for determining the velocity of blood flowing through a blood vessel or through the heart of a patient. In such a system, pulses of acoustic waves of known frequency are launched into the body of the patient along a path intersecting the stream. Due to the Doppler effect, the acoustic waves reflected by the particles in the stream are shifted in their frequency and from this frequency shift the velocity of the particles can be determined.

A system and a method for measuring the velocity of particles flowing through a sample volume according to the preambles of claim 1 and claim 7 are known from EP—A—00 14 793. In the known system, the velocity of the flow of blood particles in measured by coherent demodulation of received echo signals in two quadrature components and subsequent processing by a complex Fourier transform processor. The in-phase and quadrature signals are sampled a plurality of times and from each of these samples the velocity of the corresponding particles in the sample volume is derived. These velocity values are individually stored in a memory and can be displayed as a velocity profile on a display means. For obtaining a mean velocity of the stream, it is suggested to average the velocity values. The processing of a plurality of velocity values requires a high circuit expenditure. Moreover, the accuracy of the velocity of particles measured by the prior art system and method is relatively low.

GB—A—20 59 590 discloses an echo type range finding system as employed in a camera for the purpose of regulating the camera's automatic focusing system. In accordance with this prior art system, the distance to be measured is determined by measuring the period between transmitting an ultrasonic burst and receiving the leading edge of the reflected burst. For discriminating the reflected burst from electronic noise, the received reflected signal is rectified, continuously sampled and fed to an integrator forming the integrates of the sampled received signal voltages. The received sampled values are integrated as long as the sampled signal voltage increases between successive samples by at least a minimum rate. Thus, noise peaks of short duration can be disregarded. This reference does not relate to pulse Doppler systems for determining the velocity of particles.

Relative to this prior art, it is the object of the invention to proved an ultrasonic pulsed Doppler system in accordance with the preamble of claim 1 and a method for measuring the velocity of particles in accordance with the preamble of claim 7 permitting the measurement of the velocity of a stream of particles with a Doppler system having a smaller circuit expenditure and with higher accuracy.

According to the invention, this object is achieved by a system in accordance with the preamble of claim 1 having the features indicated in the characterizing portion thereof and by a method in accordance with the preamble of claim 7 having the features indicated in the characterizing portion thereof.

According to an underlying concept of the invention, the electrical signals corresponding to the reflected and frequency shifted acoustic waves are sampled a plurality of times each time after the launching of a pulse and the sample values are accumulated to form a combined value which is then supplied to a circuit for deriving the corresponding velocity. Different to the prior art, the sampled values are accumulated before the velocity is derived and it is only the accumulated value that is used for determining the velocity. Thus, the circuit expenditure is reduced since only a single channel is required for deriving the mean velocity from a plurality of sampled values. Furthermore, the result for the average flow velocity is more accurate than in the prior art. The reason is that the measuring error is larger in the case when a velocity value is derived from each sampled value with subsequent averaging of the velocity values than in the present invention, wherein the sampled values are first combined and then the velocity is derived from this combined value. This can be understood by taking into account that when sampling at a single instant in time after the launching of a pulse the covered sample volume is very small and the corresponding uncertainty of the velocity of the particles in this sample volume is large, whereas in the present invention the covered sample volume is larger due to the multiple sampling after the launching of a pulse and the uncertainty of the velocity is smaller.

According to claims 3 or 4, the voltage signal at the output of the accumulator can have a maximum of voltage level which is advantageous for further signal processing without saturating subsequent circuits.

According to claim 5, an extended sample volume can be completely covered without intermediate gaps, so that all the particles in the sample volume can contribute to the derived mean value for the velocity.

The invention has the further advantage that the sample volume for which the average velocity of particles is to be determined can be increased simply by taking additional samples of the reflections of each launched pulse and by accumulating these samples. This is particularly advantageous for the examination of large sample volumes like the ventricles of the heart where the length of the sample volume which is covered by taking a single sample (this length is equal to one-half of the product of the velocity of propagation of the sound wave and the duration of the pulse) is not great enough to extend across the stream of particles for which the velocities are being sought. According to an embodiment of the invention, the first of a plurality of samples can be taken at such time that its corresponding sample volume extends from the near side of the stream of scatterers, and a required number of additional samples can be taken at successively later instants of time so that their respective sample volumes combine to effectively straddle the stream. If the time between samples is equal to the duration of a pulse, the end of one sample volume will abut the beginning of the next, but the time between samples could be shortened so that the adjacent ends of the sample volumes overlap, or the time between samples could be increased so that there are gaps between the sample volumes.

According to a preferred embodiment of the invention, the accumulator is cleared after the sum or the average for all the samples associated with each launched pulse is obtained. The output of the accumulator can be applied to means for performing a Fourier analysis, such as an FFT, but if this is done, filters must be employed to attenuate the sampling transients resulting from the addition of each sample voltage and the clearing before accumulation. Filters for attenuating these sampling transients can be simplified by coupling an output sampler of the sample-and-hold type to the output of the accumulator and causing it to sample after the accumulator attains its final sum. Samples of the output of the output sampler are then applied to an FFT.

Subsequently, an embodiment of the invention is explained with reference to the drawings.

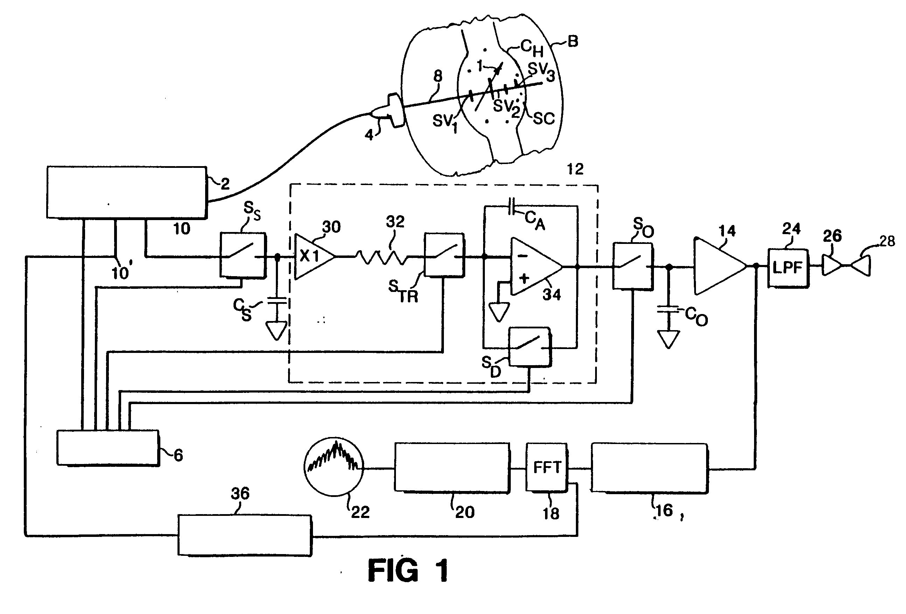

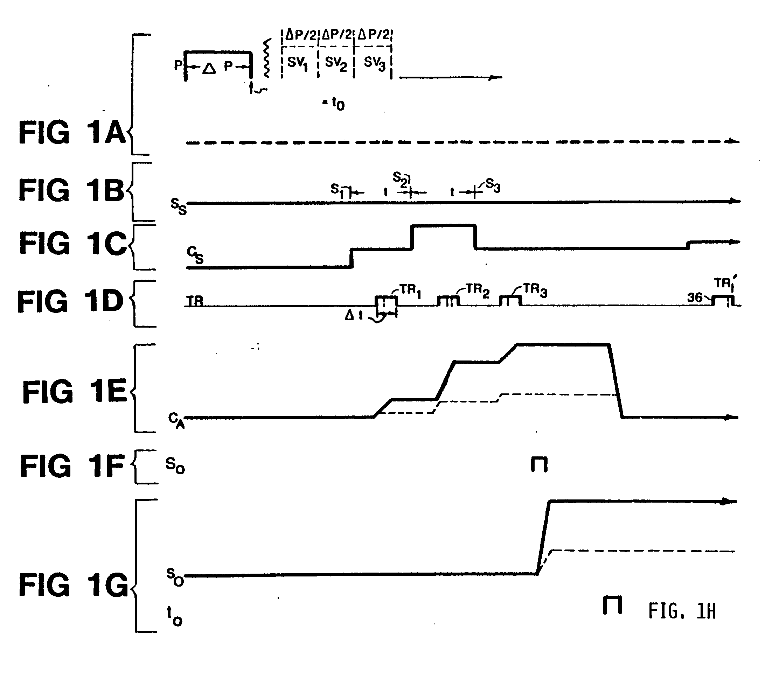

Figure 1 is a schematic diagram of one form of apparatus for carrying out the invention;

Figure 1A illustrates a launched pulse and some sample volumes;

Figure 1B illustrates a plurality of samples;

Figure 1C illustrates the voltage built up on an input capacitor;

Figure 1D illustrates the timing of a switch that transfers the voltage on the input capacitor to the accumulator;

Figure 1E illustrates the build-up of voltage by the accumulator;

Figure 1F illustrates the timing of a sample-and-hold circuit that is coupled to the output of the accumulator;

Figure 1G illustrates the output of the sample-and-hold circuit; and

Figure 1H illustrates the timing of a switch that clears the accumulator.

Reference is now made to Figure 1. The invention will now be described as it would be used to measure the velocities of blood particles flowing in the general direction of an arrow 1 through a heart chamber CH contained within the body B of a patient. The relative size of the chamber CH is exaggerated for explanatory purposes. A transmitter/receiver 2 causes a transducer 4 that is in intimate physical contact with the body B to launch pulses of a several cycles of pressure variation at a given repetition rate controlled by a timer 6 along a path 8 so as to intersect the flow of blood indicated by the arrow 1. Reflections of some of the energy in the pressure waves of the launched pulses by blood particles indicated by dots SC travel back to the transducer 4 along the path 8 and are converted therein to corresponding voltage variations. These voltage variations or signals are amplified in the transmitter/receiver 2 and appear at its output 10.

The signals at the output 10 of the transmitter/receiver 2 are applied to a sampling means herein shown as being comprised of a sampling switch $S_S$ and a sample capacitor $C_S$ connected in the order named between the output 10 and ground. The switch $S_S$ is controlled by a timer 6. The junction of the switch $S_S$ and the capacitor $C_S$ is connected to the input of an accumulator 12 which is herein shown as being a current integrator, and the output of the accumulator 12 is connected, if desired, to an accumulator output sampling means herein shown as being comprised of an output switch $S_O$, that is controlled by the timer 6, and an output capacitor $C_O$ that are connected in series in the order named between the output of the accumulator 12 and ground. An amplifier 14 is connected between the ungrounded side of the capacitor $C_O$ and a sampler 16 for an FFT analyzer 18 and a processor 20 is coupled to the output of the FFT analyzer 18 so as to prepare the signals received therefrom for display on a display means 22. If desired, the sampled signals can be listened to by connecting a low pass filter 24, an audio amplifier 26, and a loudspeaker 28 to the output of the amplifier 14.

Although a different type of accumulator could be used for storing successive samples provided by the sampling means $S_S$, $C_S$, the accumulator 12 is comprised of a buffer amplifier 30 having its input connected to the ungrounded side of the sampling capacitor $C_S$. A series circuit comprised of a resistor 32 and a transfer switch $S_{TR}$ that may be connected in either order is connected between the ouput of the buffer amplifer 30 and the inverting input of an operational amplifier 34. The non-inverting input is connected to ground. An accumulating capacitor $C_A$ and a discharge switch $S_D$ are respectively connected between the output of the amplifier 34 and its inverting input. The switches $S_{TR}$ and $S_D$ are controlled by the timer 6.

The accumulator 12 operates as follows. The buffer amplifier 30 and the resistor 32 form a constant current source that provides current with an amplitude corresponding to the voltage across the sampling capacitor $C_S$. After each closure of the sampling switch $S_S$, the transfer switch $S_{TR}$ is closed so as to permit current corresponding to the voltage across the sampling capacitor $C_S$ to flow into the accumulating capacitor $C_A$ and increase the charge thereon. It is this net charge that is related to the summation of successive sample voltages appearing on $C_S$. And, of course, the voltage at the output of the operational amplifier 34 will follow the changes in voltage across $C_A$.

If the sampling switch $S_S$ is closed only once after the launching of each pulse, as would be the case if the sample volume were long enough, the transfer function of the accumulator 12, which is equal to $\Delta t/RC$ where $\Delta t$ is the time the transfer switch $S_{TR}$ is closed, R is the ohmic value of the resistor 32 and C is the capacitance of $C_A$, could be set at such a value that the maximum voltage expected at the output of the amplifier 34 under normal operating conditions would be as large as possible without saturating subsequent circuits. But if, in accordance with this invention, the sampling switch $S_S$ is closed a plurality of times after each launched pulse so as to successively charge the sampling capacitor $C_S$ in accordance with the reflections from scatterers in the respective sample volumes $SV_1$, $SV_2$ and $SV_3$ of Figure 1, and if the transfer function remains the same, the voltage at the output of the amplifier 34 could become large enough to saturate subsequent circuits. If, for example, $\Delta t/RC=1$, the accumulator 12 becomes a summing device that simply sums the successive sample voltages appearing across $C_S$ so that the voltage at the output of the amplifier 34 would be great enough to saturate subsequent circuits. This can be avoided by reducing the transfer function $\Delta t/RC$ in any of a number of ways. One way is to couple the transfer switch $S_{TR}$ to the timer 6 in such manner as to reduce the time $\Delta t$ during which it is closed. Other ways will readily occur to one skilled in the art such as, e.g., changing the value of the resistor 32 and/or the value of the capacitor $C_A$. In any case, if the transfer function is made equal to $1/n$ where n equals the number of samples, the voltage at the output of the amplifier 34 would be an average of the samples and would not exceed the voltage of a single sample. Instead of changing the transfer function as described, means could be provided for changing the voltage to which the sampling capacitor $C_S$ is charged, e.g., by connecting different valued resistors in series with it and controlling the time during which the sampling switch $S_S$ is closed.

After all of the samples following each launched pulse have been processed as described, the timer 6 closes the switch $S_D$ so as to discharge the accumulating capacitor $C_A$ in preparation for a new series of samples.

Although the voltage appearing at the output of the accumulator 12 could be used directly, the removal of the sampling transients caused by the charge and discharge of $C_A$ is simplified by using the output sample-and-hold device $S_O$, $C_O$ that is connected between the output of the accumulator 12 and the amplifier 14. The switch $S_O$ is closed for an instant by the timer 6 after the accumulator 12 has performed its last summing operation so that the capacitor $C_O$ is charged to the voltage at the output of the accumulator 12 occurring at this time. The voltage across the capacitor $C_O$ remains until $S_O$ is closed again and is coupled via amplifier 14 to a sampler 16 that provides the samples that can be analyzed by a Fast Fourier Transform 18 so as to supply signals representing the amplitudes of discrete frequencies. A processor 20 is coupled to the output of the FFT 18 so as to derive signals representing the velocities of the various blood particles SC in such manner that they can be displayed on a cathode ray tube 22.

In the interest of simplification, only a single channel has been shown, but in order to determine the direction in which scatterers are flowing, a channel 36 similar to that just described is provided that is supplied with the signals at an output 10' of the transmitter/receiver 2 that are in phase quadrature with the signals at the output 10.

Reference is now made to Figures 1A through Figure 1H for an explanation of the operation of Figure 1. In Figure 1A, the leading edge of a pulse P that is about to be launched is shown as occurring at $t_0$. Inasmuch as the pulse P travels at a uniform velocity, Figure 1A is also a spacial plot of the position of the pulse at different times. The length of P is $\Delta P$. The timer 6 supplies a series of pulses that are t seconds apart to the sampling switch $S_S$ when the received reflections are from the scatterers at the range of interest. The successive samples $S_1$, $S_2$ and $S_3$ thus derived are shown in Figure 1B as respectively having three units, six units and three units of amplitude. As shown in Figure 1C, the voltage across $C_S$ quickly assumes and holds the value of each sample. After each of the samples $S_1$, $S_2$, and $S_3$ are taken, the timer 6 closes the transfer switch $S_{TR}$ for a period of time $\Delta t$ as shown at $S_{TR1}$, $S_{TR2}$ and $S_{TR3}$ in Figure 1D. During each closure of the switch $S_{TR}$, the capacitor $C_A$ is charged by current from the constant current source 30, 32. The current has a value proportional to the voltage across $C_S$. If the transfer founction $\Delta t/RC$ is unity, the successive samples will be summed at the output of the amplifier 34, as indicated by the solid line in Figure 1E; but if the transfer function is reduced to one-third by closing $S_{TR}$ for a shorter period $\Delta t=(1/3)RC$, the maximum output of the amplifier 34 and therefore of the accumulator 12 will be the average of the samples, or four units, as indicated by the dashed line in Figure 1E.

If the output sampler $S_O$, $C_O$ is not used, the output of the accumulator 12 is applied to the input of the sampler 16 via a filter, not shown; but if the output sampler $S_O$, $C_O$ is used, its switch $S_O$ is closed by the timer 6 after all the samples have been applied to the accumulator 12 as indicated in Figure 1F. This quickly charges the capacitor $C_O$ to the voltage at the output of the accumulator 12. As indicated in Figure 1G, this would be twelve units if $S_{TR}$ were closed for $\Delta t=RC$ seconds as indicated by the solid line, and four units if $S_{TR}$ were closed for $t=(1/3)RC$ as indicated by the dashed line. After the sampler 16 has taken a sample, the timer 6 closes the switch $S_D$ for a brief interval as indicated in Figure 1H so as to discharge $C_A$ as shown in Figure 1E before the next set of samples is received.

When the samples $S_1$, $S_2$ and $S_3$ are taken, the respective sample volumes $SV_1$, $SV_2$ and $SV_3$ are located as indicated in Figure 1A.

## Claims

1. Ultrasonic pulsed Doppler system for measuring the velocity of a stream of particles flowing through a sample volume, comprising:

a transmitter (2, 4) for transmitting ultrasound pulses of a predetermined carrier frequency into the sample volume during each of successive sending/receiving periods,

a receiver (2, 4) for receiving ultrasound signals reflected from the streaming particles in the sample volume and being frequency shifted relative to the carrier frequency during each of the sending/receiving periods and for converting the reflected signals into electrical output signals,

a sampling circuit ($S_S$, $C_S$) for sampling the electrical output signals to derive sampled values,

a signal processing means (12 to 20) for deriving from the sampled values the velocity of particles flowing through the sample volume, and

a control circuit (6) coupled to the sampling circuit ($S_S$, $C_S$), characterized in that

the control circuit activates the sampling circuit ($S_S$, $C_S$) several times during each sending/receiving period to provide several sampled values,

said signal processing means comprises an accumulator (12) coupled to the sampling circuit ($S_S$, $C_S$) for accumulating the sampled values during a sending/receiving period to provide an accumulated value, and

said signal processing means further comprises a processing circuit (16, 18, 20) coupled to the accumulator (12) for deriving said velocity of particles from the accumulated value.

2. System according to claim 1, characterized in that the accumulator (12) provides an accumulated value corresponding to the sum of the sampled values.

3. System according to claim 1, characterized in that the accumulator (12) provides an accumulated value corresponding to the average value of the sampled values.

4. System according to claim 1, characterized in that the accumulator (12) provides an accumulated value corresponding to a weighted average of the sampled values.

5. System according to any of the preceding claims, characterized in that the control circuit (6) activates the sampling circuit ($S_S$, $C_S$) at such instants of time that the time intervals between successive sampling instants substantially correspond to the duration of the ultrasonund pulse transmitted in this sending/receiving period.

6. System according to any of the preceding claims, characterized in that the processing circuit comprises a circuit (18, 20) for performing a Fourier analysis of the accumulated values.

7. Method for measuring the velocity of particles flowing through a sample volume by means of an ultrasonic pulsed Doppler system, comprising the steps of

launching ultrasound pulses of a predetermined carrier frequency,

receiving reflections of each launched ultrasound pulse,

obtaining samples of the reflections of each launched pulse, and

processing said samples for deriving the velocity of particles, characterized in that a plurality of samples are obtained by successively activating a sampling circuit a plurality of times after each launching of a pulse, and that the step of processing said samples comprises the steps of

accumulating said samples so as to derive an accumulated sample, and

processing said accumulated sample for deriving said velocity of particles.

8. Method as claimed in claim 7, characterized in that the samples are weighted before they are accumulated.

9. Method as claimed in claim 7 or 8, characterized in that the step of processing said accumulated sample comprises the performing of a Fourier analysis of said accumulated sample.

10. Method as claimed in one of the claims 7 to 9, characterized in that the samples of the reflections of each launched pulse at respectively successive times are obtained such that each sample is of the reflections of particles contained in a different sample volume, which is defined by the length of the Doppler pulse along the line of propagation of the pulse, to thereby obtain a velocity distribution of the particles along the path of the Doppler pulse and within a volume having a length along the line of propagation that is greater than the length of the sample volume.

## Patentansprüche

1. Ultraschall-gepulstes Dopplersystem zum Messen der Geschwindigkeit eines durch ein Meßvolumen fließenden Teilchenstromes, mit:

einem Sender (2, 4) zum Senden von Ultraschallpulsen mit einer vorbestimmten Trägerfrequenz in das Meßvolumen während einer jeden Periode von aufeinanderfolgenden Sende-/Empfangs-Perioden,

einem Empfänger (2, 4) zum Empfangen von Ultraschallsignalen in jeder der Sende-/Empfangs-Perioden, die von den strömenden Teilchen in dem Meßvolumen reflektiert werden und die frequenzmäßig gegenüber der Trägerfrequenz versetzt sind und zum Umwandeln der reflektierten Signale in elektrische Ausgangssignale,

einer Abtastschaltung ($S_S$, $C_S$) zum Abtasten der elektrischen Ausgangssignale, um abgetastete Werte zu erhalten,

einer Signalverarbeitungseinrichtung (12—20) zum Ableiten der Geschwindigkeit der durch das Meßvolumen fließenden Teilchen von den abgetasteten Werten, und

einer Steuerschaltung, (6) die an die Abtastschaltung ($S_S$, $C_S$) angeschlossen ist, dadurch gekennzeichnet, daß

die Steuerschaltung die Abtastschaltung ($S_S$, $C_S$) mehrere Male während einer jeden Sende-/Empfangs-Periode betätigt, um eine Mehrzahl von abgetasteten Werten zu erzeugen,

die Signalverarbeitungseinrichtung einen Akkumulator (12) aufweist, der an die Abtastschaltung

(S$_S$, C$_S$) angeschlossen ist, um die abgetasteten Werte während einer Sende-/Empfangs-Periode zum Erzeugen eines akkumulierten Wertes zu akkumulieren, und

die Signalverarbeitungseinrichtung ferner eine Verarbeitungsschaltung (16, 18, 20), aufweist, die an den Akkumulator (12) angeschlossen ist, um die Geschwindigkeit der Teilchen von dem akkumulierten Wert abzuleiten.

2. System nach Anspruch 1, dadurch gekennzeichnet, daß der Akkumulator (12) einen akkumulierten Wert erzeugt, der der Summe der abgetasteten Werte entspricht.

3. System nach Anspruch 1, dadurch gekennzeichnet, daß der Akkumulator (12) einen akkumulierten Wert erzeugt, der dem Durchschnittswert der abgetasteten Werte entspricht.

4. System nach Anspruch 1, dadurch gekennzeichnet, daß der Akkumulator einen akkumulierten Wert erzeugt, der einem gewichteten Mittel der abgetasteten Werte entspricht.

5. System nach einem der vorhergehenden Ansprüche, dadurch gekennzeichnet, daß die Steuerschaltung (6) die Abtastschaltung (S$_S$, C$_S$) zu derartigen zeitlichen Momenten aktiviert, daß die Zeitintervalle zwischen den aufeinanderfolgenden Abtastmomenten im wesentlichen der Dauer des in dieser Sende-/Empfangs-Periode gesendeten Ultraschallpulses entsprechen.

6. System nach einem der vorhergehenden Ansprüche, dadurch gekennzeichnet, daß die Verarbeitungsschaltung eine Schaltung (18, 20) zum Durchführen einer Fourier-Analyse der akkumulierten Werte aufweist.

7. Verfahren zum Messen der Geschwindigkeit von durch ein Meßvolumen fließenden Teilchen mittels eines Ultraschall-gepulsten Dopplersystemes, mit den Verfahrensschritten des:

Aussendens von Ultraschallpulsen einer vorbestimmten Trägerfrequenz,

Empfangens von Reflexionen eines jeden ausgesendeten Ultraschallpulses,

Erhaltens von Abtastwerten der Reflexionen eines jeden ausgesandten Pulses, und

Verarbeitens der Abtastwerte zum Ableiten der Geschwindigkeit der Teilchen, durch gekennzeichnet, daß eine Mehrzahl von Abtastwerten durch aufeinanderfolgendes Aktivieren einer Abtastschaltung mehrere Male nach jedem Aussenden eines Pulses erhalten werden, und

der Verfahrensschritt des Verarbeitens der Abtastwerte folgende Schritte aufweist:

Akkumulieren der Abtastwerte zum Ableiten eines akkumulierten Abtastwertes, und

Verarbeiten des akkumulierten Abtastwertes zum Ableiten der Geschwindigkeit der Teilchen.

8. Verfahren nach Anspruch 7, dadurch gekennzeichnet, daß die Abtastwerte vor ihrem Akkumulieren gewichtet werden.

9. Verfahren nach Anspruch 7 oder 8, dadurch gekennzeichnet, daß der Schritt des Verarbeitens des akkumulierten Abtastwertes das Durchführen einer Fourier-Analyse des akkumulierten Abtastwertes umfaßt.

10. Verfahren nach einem der Ansprüche 7—9, dadurch gekennzeichnet, daß die Abtastwerte der Reflexionen eines jeden ausgesendeten Pulses zu jeweils aufeinanderfolgenden Zeitpunkten derart erhalten werden, daß der Abtastwert von den Reflexionen von in unterschiedlichen Meßvolumen enthaltenen Teilchen stammt, wobei das Meßvolumen durch die Länge des Dopplerpulses längs der Ausbreitungslinie des Pulses festgelegt ist, um eine Geschwindigkeitsverteilung der Teilchen längs des Weges des Dopplerpulses und innerhalb eines Volumens mit einer Länge längs der Ausbreitungslinie, die größer als die Länge des Meßvolumens ist, zu erhalten.

**Revendications**

1. Système Doppler à impulsions ultrasoniques pour mesurer la vitesse d'un courant de particules s'écoulant au travers d'un volume d'échantillonnage, comprenant:

un émetteur (2, 4) pour émettre des impulsions ultrasoniques d'une fréquence porteuse prédéterminée dans le volume d'échantillonnage pendant chacune des périodes d'émission/réception successives,

un récepteur (2, 4) pour recevoir des signaux ultrasoniques réfléchis par les particules de l'écoulement dans le volume d'échantillonnage et décalés en fréquence par rapport à la fréquence porteuse pendant chacune des périodes d'émission/réception, et pour convertir les signaux réfléchis en signaux électriques de sortie,

un circuit d'échantillonnage (S$_S$, C$_S$) pour échantillonner les signaux électriques de sortie pour en dériver des valeurs échantillonnées,

un moyen de traitement de signaux (12 à 20) pour obtenir à partir des valeurs échantillonnées la vitesse des particules s'écoulant au travers du volume d'échantillonnage, et

un circuit de commande (6) relié au circuit d'échantillonnage (S$_S$, C$_S$), caractérisé en ce que:

le circuit de commande excite le circuit d'échantillonnage (S$_S$, C$_S$) plusieurs fois pendant chaque période d'émission/réception afin de produire plusieurs valeurs échantillonnées,

ledit moyen de traitement de signaux comprend un accumulateur (12) relié au circuit d'échantillonnage (S$_S$; C$_S$) pour accumuler les valeurs échantillonnées pendant une période d'émission/réception afin de produire une valeur accumulée, et

ledit moyen de traitement de signaux comprend en outre un circuit de traitement (16, 18, 20) relié à l'accumulateur (12) pour déterminer ladite vitesse de particules à partir de la valeur accumulée.

2. Système selon la revendication 1, caractérisé en ce que l'accumulateur (12) produit une valeur accumulée correspondant à la somme des valeurs échantillonnées.

3. Système selon la revendication 1, caractérisé en ce que l'accumulateur (12) produit une valeur accumulée correspondant à la valeur moyenne des valeurs échantillonnées.

4. Système selon la revendication 1, caractérisé en ce que l'accumulateur (12) produit une valeur accumulée correspondant à la moyenne pondérée des valeurs échantillonnées.

5. Système selon une quelconque des revendications précédentes, caractérisé en ce que le circuit de commande (6) excite le circuit d'échantillonnage ($S_s$, $C_s$) à des instants tels que les intervalles de temps entre des instants d'échantillonnage successifs correspondent sensiblement à la durée de l'impulsion ultrasonique transmise dans cette période d'émission/réception.

6. Système selon une quelconque des revendications précédentes, caractérisé en ce que le circuit de traitement comprend un circuit (18, 20) pour effectuer une analyse de Fourier des valeurs accumulées.

7. Procédé pour mesurer la vitesse de particules s'écoulant au travers d'un volume d'échantillonnage au moyen d'un système Doppler à impulsions ultrasoniques, comprenant les étapes consistant à:

émettre des impulsions ultrasoniques d'une fréquence porteuse prédéterminée,

recevoir des réflexions de chaque impulsion ultrasonique émise,

obtenir des échantillons des réflexions de chaque impulsion émise, et

traiter lesdits échantillons pour en dériver la vitesse des particules, caractérisé en ce que:

une pluralité d'échantillons sont obtenus par excitation successive d'un circuit d'échantillonnage une pluralité de fois après chaque émission d'une impulsion, et en ce que l'étape de traitement desdits échantillons comprend les étapes consistant à:

accumuler lesdits échantillons pour en dériver un échantillon accumulé, et

traiter ledit échantillon accumulé pour en dériver ladite vitesse de particules.

8. Procédé tel que revendiqué dans la revendication 7, caractérisé en ce que les échantillons sont pondérés avant qu'ils soient accumulés.

9. Procédé tel que revendiqué dans la revendication 7 ou 8, caractérisé en ce que l'étape de traitement dudit échantillon accumulé comprend l'exécution d'une analyse de Fourier dudit échantillon accumulé.

10. Procédé tel que revendiqué dans une des revendications 7 à 9, caractérisé en ce que les échantillons des réflexions de chaque impulsion émise à des instants respectivement successifs sont obtenus de telle sorte que chaque échantillon concerne les réflexions de particules contenues dans un volume d'échantillonnage différent, qui est défini par la longueur de l'impulsion Doppler le long de la ligne de propagation de l'impulsion, pour obtenir ainsi une distribution de vitesse des particules le long du trajet de l'impulsion Doppler et à l'intérieur d'un volume ayant, le long de la ligne de propagation, une longueur qui est supérieure à la longueur du volume d'échantillonnage.

**FIG 1**

EP 0 173 243 B1

FIG 1A
FIG 1B
FIG 1C
FIG 1D
FIG 1E
FIG 1F
FIG 1G

FIG. 1H

EP 0 173 243 B1